# EUROPEAN PATENT APPLICATION

(11) **EP 3 427 759 A1**
(43) Date of publication of application: **16.01.2019**
(21) Application number: 17890843.0
(22) Date of filing: 02.06.2017
(51) Int. Cl.: A61L 2/14, A61L 2/20, A61L 2/26

(54) **LED MONITORING SYSTEM, PLASMA STERILIZATION DEVICE PROVIDED WITH SAME, AND STERILANT CASSETTE INCLUDING HYDROGEN PEROXIDE**

(71) Applicant: Zeronitec Co., Ltd., Bucheon-si, Gyeonggi-do 14502 (KR)
(72) Inventor: KIM, Myung Sun, Songpa-gu, Seoul 05555 (KR); LEE, Han, Gi, Hanam-si, Gyeonggi-do 12945 (KR)
(74) Representative: Glawe, Delfs, Moll
(86) International application number: PCT/KR2017/005806
(87) International publication number: WO 2018/221765

(57) **Abstract**

Provided are a light emitting diode (LED) monitoring system included in a plasma sterilizer for sterilizing, in a vacuum, objects such as medical instruments, the plasma sterilizer having the LED monitoring system, and a sterilant cassette containing hydrogen peroxide, and more particularly, an LED monitoring system which uses the on/off and color change of a plurality of LED lamps to indicate the progress of sterilization through the on/off and color change of the LED lamps, a plasma sterilizer having the LED monitoring system, and a sterilant cassette containing hydrogen peroxide in which a plurality of ampules for supplying a sterilant are fixed upright, the LED monitoring system, the plasma sterilizer and the sterilant cassette being provided so that the sterilization and disinfection process can be identified at a glance, the sterilant cassette containing the hydrogen peroxide can be easily transported and replaced, the sterilant can be injected in a fixed quantity, and productivity can be improved (a sterilization cycle can be shortened) by simplifying a sterilant injection process.

## Description

### [Technical Field]

The present disclosure relates to a light emitting diode (LED) monitoring system included in a plasma sterilizer for sterilizing, in a vacuum, objects such as medical instruments, the plasma sterilizer having the LED monitoring system, and a sterilant cassette containing hydrogen peroxide, and more particularly, to an LED monitoring system which uses the on/off and color change of a plurality of LED lamps to indicate the progress of sterilization through the on/off and color change of the LED lamps, a plasma sterilizer having the LED monitoring system, and a sterilant cassette containing hydrogen peroxide in which a plurality of ampules for supplying a sterilant are fixed upright, the LED monitoring system, the plasma sterilizer and the sterilant cassette being provided so that the sterilization and disinfection process can be identified at a glance, the sterilant cassette can be easily transported and replaced, the sterilant can be injected in a fixed quantity, and productivity can be improved (a sterilization cycle can be shortened) by simplifying a sterilant injection process.

### [Background Art]

Generally, endoscopes, medical instruments for surgical operations, etc. are sterilized and disinfected using a sterilizer to kill microorganisms existing on surfaces and lumens. Examples of such a sterilizer include a high-pressure steam sterilizer (steam sterilization), an ethylene oxide (EO) gas sterilizer, and a plasma sterilizer.

The high-pressure steam sterilizer performs sterilization using steam at high pressure and thus is not suitable for sterilization of medical instruments vulnerable to high temperature, humidity and/or high pressure.

The ethylene oxide gas sterilizer uses a toxic ethylene oxide gas. Thus, toxic residues may remain on medical instruments. Accordingly, a separate residual gas removal process for removing toxic gases must be performed. This complicates the sterilization process, thereby reducing productivity (long sterilization time) and increasing the sterilization cost.

On the other hand, the plasma sterilizer can perform sterilization in a vacuum by using plasma and can easily decompose a sterilant into environmentally harmless substances by using the plasma.

In the plasma sterilizer, a sterilant is fed by pouring the sterilant into an ampule (container) containing the sterilant after a user removes a cap of the ampule. However, this sterilant supply method lowers work efficiency, and the sterilant can be unintentionally leaked in the process of mounting the ampule in a mounting unit (holder) of the sterilizer.

In addition, in the plasma sterilizer, if one sterilant ampule is used, the user has to replace the ampule frequently because the ampule can be used only a few times. Since the ampule has to be pierced during the sterilization process, it is smeared with toxic chemicals. Therefore, the user has to be very careful when replacing the ampule.

Furthermore, since the conventional plasma sterilizer does not have a display device through which the sterilization process and information can be easily identified at a glance, the sterilization process should be selected and identified using only a touch screen for operation control

### [Disclosure]

### [Technical Problem]

Aspects of the present disclosure provide a light emitting diode (LED) monitoring system which uses the on/off and color change of a plurality of LED lamps to enable a user to easily identify a sterilization and disinfection process at a glance and rapidly cope with the situation and a plasma sterilizer having the LED monitoring system.

Aspects of the present disclosure also provide a plasma sterilizer and a sterilant cassette containing hydrogen peroxide, in which a plurality of sterilant ampules can be mounted at a time using the sterilant cassette containing the hydrogen peroxide, ampule piercing for sterilant injection can be performed safely, and, in particular, a cassette body for fixing the ampules upright between an upper body and a lower body is provided to enable the fixed quantity injection and complete use of a sterilant.

Aspects of the present disclosure also provide a plasma sterilizer and a sterilant cassette containing hydrogen peroxide, in which a plurality of fitting holes which are arranged radially and into which neck portions connected to upper parts of ampules are fitted and a fitting groove which is formed in a ring shape and into which fitting portions connected to lower parts of the ampules are fitted are respectively provided in circular plate-shaped upper and lower bodies to make it easy to replace the ampules using rotation of a cassette body.

Aspects of the present disclosure also provide a plasma sterilizer and a sterilant cassette containing hydrogen peroxide, in which a folding portion is provided between wings of adjacent ampules so as to enable the ampules to be easily mounted in and removed from a cassette body and to be bent radially and easily arranged in a circle.

### [Technical Solution]

According to an aspect of the present disclosure, there is provided a light emitting diode (LED) monitoring system which is mounted in a plasma sterilizer for sterilizing objects in a vacuum and indicates the progress of sterilization using the on/off, color change or both of a plurality of LED lamps.

According to another aspect of the present disclosure, there is provided a plasma sterilizer for sterilizing objects in a vacuum. The plasma sterilizer includes a sterilant cassette containing hydrogen peroxide which is mounted in a mounting unit; a vacuum chamber in which the objects are received; a sterilant injector which sucks a sterilant from ampules of the sterilant cassette and supplies the sucked sterilant to the vacuum chamber, and an LED monitoring system which indicates the progress of sterilization using the on/off, color change or both of a plurality of LED lamps.

According to another aspect of the present disclosure, there is provided a sterilant cassette containing hydrogen peroxide and mounted in a plasma sterilizer for sterilizing objects in a vacuum. The sterilant cassette containing the hydrogen peroxide includes a plurality of ampules in which a sterilant is stored; and a cassette body which is composed of an upper body to which upper ends of the ampules are fixed and a lower body to which lower ends of the ampules are fixed so as to fix the ampules upright between the upper body and the lower body.

In addition, each of the ampules in the sterilant cassette containing the hydrogen peroxide is composed of a body portion which has a sterilant storage space, a neck portion which has a reducing width and is connected to an upper side of the body portion and a fitting portion which is connected to a bottom surface of the body portion, and the upper body and the lower body have circular plate shapes corresponding to each other.

The upper body includes a plurality of fitting holes whose lower surfaces are open along a rim, which are arranged radially and to which the neck portions are fitted, and the lower body includes a fitting groove which is formed in a ring shape to have an upper surface open along a rim and into which the fitting portions are fitted.

### [Advantageous Effects]

A light emitting diode (LED) monitoring system and a sterilizer having the same according to the present disclosure display the progress of sterilization and disinfection using the on/off and color change of a plurality of LED lamps. Therefore, a user can easily identify various errors or information regarding the disinfection and sterilization process at a glance in real time and rapidly cope with the situation.

In a plasma sterilizer and a sterilant cassette containing hydrogen peroxide according to the present disclosure, a plurality of ampules are arranged upright in a circle on a cassette body. Therefore, the ampoules can be easily replaced by rotation of the cassette body, a sterilant can be injected in a fixed quantity and used completely, and there is no risk of the sterilant leaking out of the ampules after being used. In addition, it is easy to replace the sterilant cassette containing the hydrogen peroxide and easy to mount the ampoules in the sterilant cassette containing the hydrogen peroxide.

### [Description of Drawings]

FIG. 1 is an enlarged view of a light emitting diode (LED) monitoring system according to the present disclosure;
FIGS. 2 and 3 are an external perspective view and a conceptual diagram of a major part of a plasma sterilizer according to the present disclosure;
FIGS. 4 and 5 are a coupled perspective view and an exploded perspective view of a sterilant cassette containing hydrogen peroxide according to the present disclosure; and
FIG. 6 is a plan view of a fixing medium of a cassette body according to the present disclosure.

### [Mode for Invention]

The present disclosure may be variously modified and embodied in many different forms, and aspects (or embodiments) are to be specifically described therein. However, the present disclosure should not be construed as limited to specific disclosure forms, and the spirit and scope of the invention should be understood as incorporating various modifications, additions and substitutions.

Wherever possible, the same reference numerals, in particular, the reference numerals in which a two-digit number and one-digit number are the same or a two-digit number, one-digit number and letters of the alphabet are the same, will be used throughout the drawings and the description to refer to the same or like members. Unless otherwise stated, the members designated by respective reference numerals in the drawings are regarded as members based on such standards.

In the drawings, elements are expressed exaggeratingly larger (or thicker) or smaller (or thinner) in size or thickness or expressed in a simplified form for ease of understanding. However, it should not be construed as limiting the scope of protection of the present disclosure.

The terminology used herein is for the purpose of describing particular aspects (or embodiments) only and is not intended to be limiting.

As used herein, the singular forms are intended to include the plural forms as well, unless the context clearly indicates otherwise.

It will be further understood that the terms "comprises" and/or "comprising," or "includes" and/or "including" when used in this specification, specify the presence of stated features, numbers, steps, operations, elements, components or combinations thereof, but do not preclude the presence or addition of one or more other features, numbers, steps, operations, elements, components or combinations thereof.

Unless otherwise defined, all terms used herein, including technical or scientific terms, have the same meanings as those generally understood by those with ordinary knowledge in the field of the art to which the present disclosure belongs. Such terms as those defined in generally used dictionaries are to be interpreted as having meanings equivalent to the contextual meanings in the relevant field of art, and are not to be interpreted as having ideal or excessively formal meanings unless clearly defined as having such in the present application.

It will be understood that the terms first, second, etc. may be used herein to describe different elements, and the sequence of formation thereof does not matter. These terms may be differently described in the detailed description and the claims of the present disclosure.

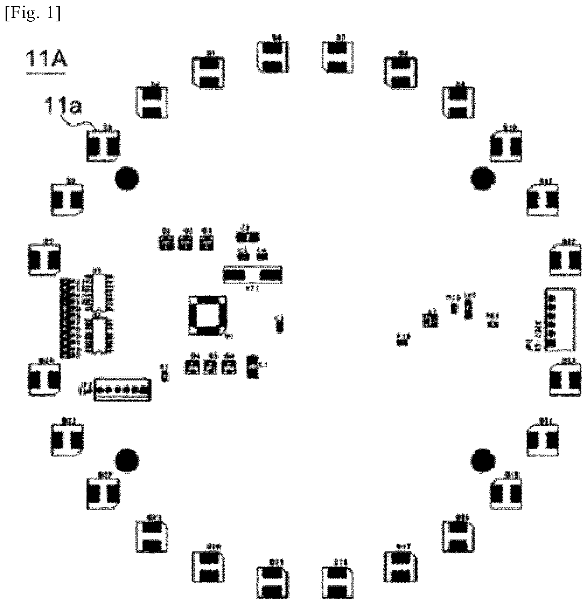

In describing a light emitting diode (LED) monitoring system, a plasma sterilizer having the same, and a sterilant cassette containing hydrogen peroxide according to the present disclosure, if an inexact and approximate standard of direction is specified with reference to FIGS. 1, 2 and 4 for the sake of convenience, the direction in which gravity acts is regarded as a downward direction, and then upward, downward, right and left directions are determined as viewed in the drawings. Unless otherwise stated, directions are also specified and described based on this standard in the detailed description and the claims of the present disclosure relating to other drawings.

Hereinafter, an LED monitoring system 11A, a plasma sterilizer having the same, and a sterilant cassette C containing hydrogen peroxide according to the present disclosure will be described with reference to the accompanying drawings.

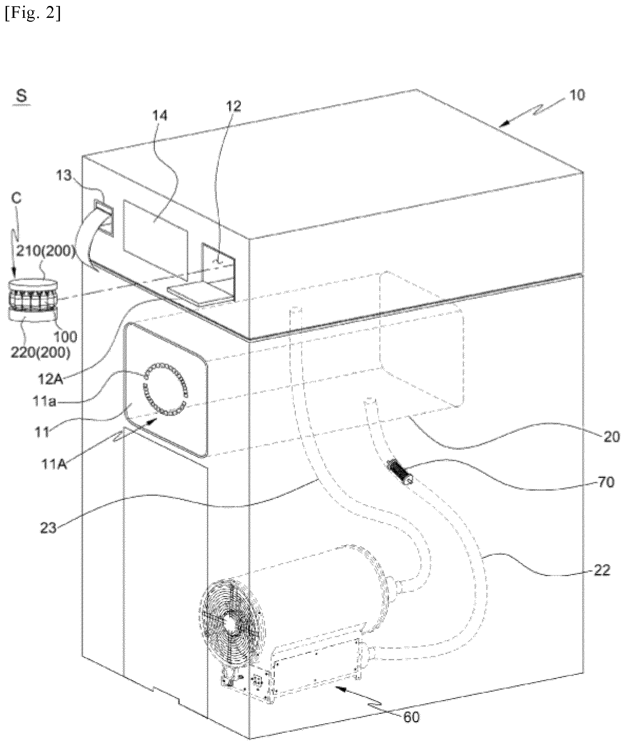

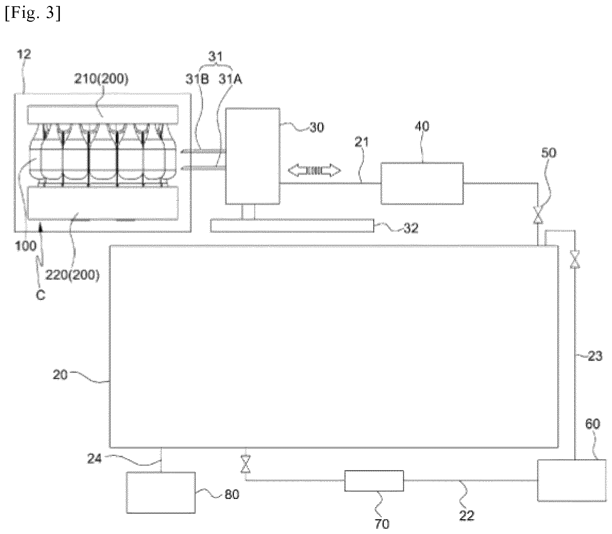

First, the LED monitoring system 11a and the plasma sterilizer S having the same according to the present disclosure will be described with reference to FIGS. 2 and 3.

The plasma sterilizer S includes, inside a main body 10, a vacuum chamber 20 which receives objects to be sterilized such as endoscopes and medical instruments for surgical operations. The vacuum chamber 20 is kept airtight with a sealed door 11.

The vacuum chamber 20 may be configured as a chamber for performing low-temperature plasma sterilization. To this end, a plasma generator (not illustrated) for generating plasma in a vaporized sterilant is connected or installed on one side.

In addition, the vacuum chamber 20 is connected to a sterilant supply line 21 on which a sterilant injector 30, a vaporizer 40 for vaporizing an injected sterilant and an open/close value 50 for supplying the vaporized sterilant to the vacuum chamber 20 are installed, connected to blowing lines 22 and 23 on which a hot air circulating dry system 70 is installed outside the vacuum chamber, and connected to a vacuum intake line 24 on which a vacuum pump 80 for keeping the vacuum chamber 20 in a vacuum is installed outside the vacuum chamber 20.

Although not illustrated in the drawings, the vacuum chamber 20 may be configured as a chamber for performing plasma sterilization. To this end, the plasma generator (not illustrated) for generating plasma in a vaporized sterilant may be connected or installed on one side. On the sterilant supply line 21 connecting the sterilant injector 30, the vaporizer 40 and the open/close valve 50, a flow controller for controlling a flow rate of a sterilant, a quantity check sensor for measuring the flow rate of the sterilant, and a metering cylinder for supplying the sterilant in a fixed quantity may be further installed.

The sterilant suction device 30 sucks the sterilant from ampules 100 of the sterilant cassette C mounted in a mounting unit 12 and supplies the sucked sterilant to the vacuum chamber 20. The sterilant suction device 30 includes a needle unit 31 formed in a horizontal direction and a moving unit 32 which moves the needle unit 31 back and forth.

The moving unit 32 is a known unit using various motors and/or rails. The moving unit 32 moves the needle unit 31 toward the mounting unit 12 to pierce the upright and fixed ampules 100 in a lateral direction, suck the sterilant stored in sterilant storage spaces, and supply the sucked sterilant to the vaporizer 40.

The needle unit 31 sucks the sterilant by using needle members having pointed tips so as to pierce body portions 110 of the ampules 100. The number of needle members is not limited, but when one or a plurality of needle members suck the sterilant at the same time, the suction of the sterilant may not be performed smoothly due to a pressure change inside the sterilant storage spaces 111.

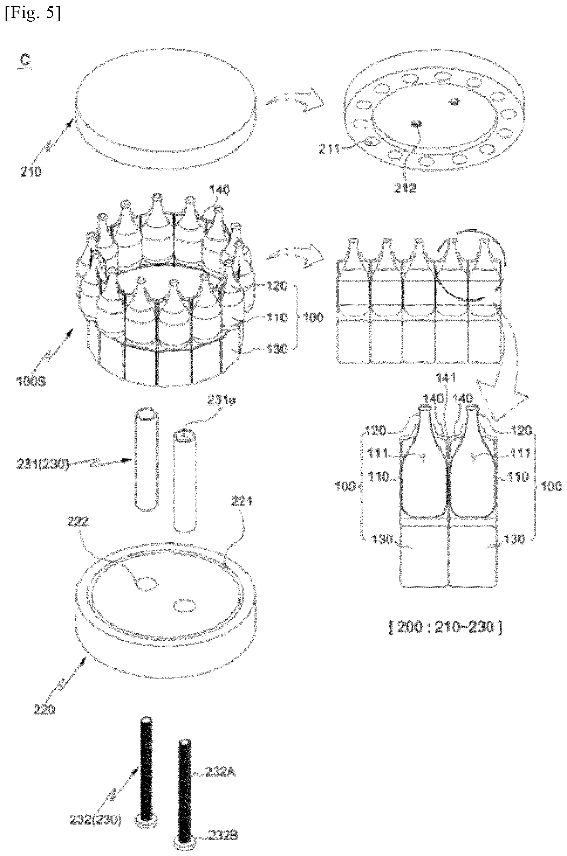

That is, since the sterilant storage spaces 111 are sealed in the ampules 100 as illustrated in FIG. 5, they are in a vacuum when the sterilant is sucked. Thus, as the pressure inside the ampules 100 is reduced, the suction of the sterilant is not smoothly performed.

To prevent the above problem, as illustrated in FIG. 3, the needle unit 31 of the present disclosure is composed of a pair of needle members 31A and 31B which are vertically spaced apart from each other by a predetermined distance. The distance between the upper needle member 31B and the lower needle member 31A corresponds to the vertical height of the ampules 100 (more strictly, the body portions 110) such that the needle members simultaneously pierce lower and upper ends of the ampules 100, respectively. Here, when the lower needle member 31A sucks the sterilant of the ampules 100, the upper needle member 31B injects air into the ampules 100.

Therefore, since external air is introduced into the ampules 100 through the upper needle member 31B according to the amount of pressure inside the ampules 100 which is reduced by the amount of sterilant sucked by the lower needle member 31A, a change in the pressure inside the ampules 100 is prevented, which enables the smooth suction of the sterilant into the lower needle member 31A.

Such simultaneous sterilant suction and air injection not only facilitate the suction of the sterilant but also has an effect of supplying an appropriate amount of sterilant to the vacuum chamber 20.

Although not illustrated in the drawings, the sterilant suction device 30 may include a suction pump for providing suction pressure and a flow control valve or a flow controller for controlling the flow rate of the sterilant.

The hot air circulating dry system 60 includes a blowing fan or the like for forcibly circulating air by blowing the air. The hot air circulating dry system 60 blows air into the first blowing line 22 so that the air is introduced into the vacuum chamber 20 while sucking the air inside the vacuum chamber 20 into the second blowing line 23 so that the air is forcibly circulated in the vacuum chamber 20 to dry objects to be sterilized. In this case, a heating unit 70 for heating blown air is embedded or connected to the first blowing line 22 so that hot air can be introduced into the vacuum chamber 20.

In addition, open/close valves (not indicated) for passing or blocking the air being circulated are provided on the first and second blowing lines 22 and 23.

The heating unit 70 may be formed in the form of a helically twisted hot wire coil and embedded in the first blowing line 22 or may be formed as a heater having a helically twisted hot wire coil and connected to the first blowing line 22.

In the plasma sterilizer S configured as described above, when medical instruments are placed in the vacuum chamber 20, the hot air circulating dry system 60 operates to dry the objects to be sterilized by using hot air forcibly circulated in the vacuum chamber 20.

Upon completion of the drying, the vacuum pump 80 operates to keep the vacuum chamber 20 in a vacuum. While the vacuum chamber is kept in a vacuum, the sterilant injector 30, the vaporizer 40 and the open/close valve 50 operate to sterilize and dry the medical instruments through the supply of the sterilant and the generation of plasma.

In order to enable injection of a sterillant such as hydrogen peroxide to sterilize and disinfect medical instruments, the sterilant mounting unit 12 is provided on a side of the main body 10 of the plasma sterilizer S.

Reference numeral 12A indicates an open/close door for opening/closing the sterilant mounting unit 12. Reference numeral 13 indicates an output unit which prints and outputs disinfection-related information. Reference numeral 14 indicates a touch screen which makes it possible to control and manipulate a series of sterilization and disinfection processes.

The plasma sterilizer S further includes the LED monitoring system 11A which enables a user to easily identify and monitor the sterilization and disinfection processes in real time.

In the drawings, the LED monitoring system 11A consisting of a plurality of LED lamps 11a is installed on the sealed door 12A. However, the present disclosure is not limited to this case.

By using the on/off, color change, or both of the LED lamps 11a, the LED monitoring system 11A displays which sterilization and disinfection process is now being performed, how much time is left to complete the sterilization and disinfection processes, and various errors occurring during the sterilization and sterilization processes.

For example, during the normal operation of the plasma sterilizer S, all of the LED lamps 11a are illuminated in green. As the steriliazation and disinfection processes proceed, the LED lamps 11a are sequentially turned off one by one to indicate the progress and the remaining time. When an error occurs during the sterilization and disinfection processes, the LED lamps 11a turn red to indicate the occurrence of the error. When the sterilization and disinfection processes are completed, the LED lamps 11a turn white to indicate the completion of the sterilization and disinfection processes.

The plasma sterilizer S may further include an audio output unit (not illustrated) which is linked with the LED monitoring system 11A to output the progress of the sterilization and disinfection processes, the occurrence of various errors, a sterilization and disinfection completion signal, etc. through an alarm or voice.

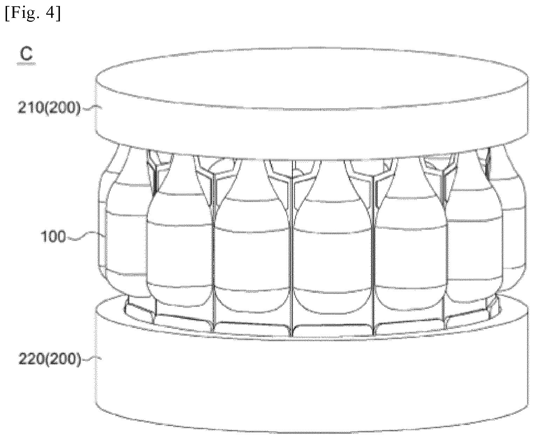

The sterilant cassette C containing the hydrogen peroxide will now be described with reference to FIGS. 4 through 6. The sterilant cassette C enables the ampules 100 storing a sterilant to be simultaneously mounted in the mounting unit 12 of the plasma sterilizer S configured as described above and enables the ampules 100 to be replaced through rotation.

Referring to FIGS. 4 and 5, the sterilant cassette C containing the hydrogen peroxide according to the present disclosure largely includes a plurality of ampules 100 which store a sterilant and a cassette body 200 in which the ampules 100 are mounted upright.

The ampules 100 are made of a synthetic resin such as soft plastic and are each composed of the body portion 110 having the sterilant storage space 111 for storing the sterilant, a neck portion 120 connected to an upper side of the body portion 110 to seal the sterilant storage space 111, a fitting portion 130 connected to a bottom surface of the body portion 110, and wings 140 protruding from both sides of the body portion 110 and connected to other ampules 100.

The body portion 110 and the neck portion 120 are integrally formed as a single tubular member. The body portion 110 and the neck portion 120 are only an arbitrary division made for the purpose of distinguishing the neck portion 120 inserted and fixed into each upper fitting hole 211 of the cassette body 200 due to a difference in thicknesses of the body portion 110 and the neck portion 120 as will be described later. The body portion 110 and the neck portion 120 are one member having the same sterilant storage space 111.

The body portion 110 and the neck portion 120 as a whole are formed in the shape of a water droplet that is narrow at the top and wide at the bottom and include the sterilant storage space 111. After the sterilant is stored, an upper opening of the neck portion 120 is sealed by, e.g., thermal bonding to keep the sterilant sealed.

The reason why the body portion 110 and the neck portion 120 are limited to the water droplet shape is to make the level of the sterilant in the sterilant storage space 111 be lowered by self-weight when the needle unit 31 (see FIG. 3) of the sterilant injector 30 injects the sterilant by laterally piercing the body portion 110 because the ampules 100 of the present disclosure are fixed in an upright position to the cassette body 200 as will be described later. This enables the fixed quantity injection and complete use of the sterilant stored in one ampule 100.

The fitting portion 130 connected to the bottom surface of the body portion 110 may be shaped like a thin flat plate integrally formed with the body portion 110 without having an internal space connected to the sterilant storage space 111, unlike the neck portion 120.

Upper ends, i.e., the neck portions 120 of the ampules 100 configured as described above are fitted and fixed into the fitting holes 211 of an upper body 210 to be described later, and lower ends, i.e., the fitting portions 130 of the ampules 100 are fitted and fixed into a fitting groove of a lower body 220. Accordingly, the ampules 100 are mounted and fixed upright between the upper body 210 and the lower body 220 of the cassette body 200.

The wings 140 make it easy to transport and store the ampules 100 and possible to simultaneously mount the ampules 100 on the cassette body 200 by connecting adjacent ampules 100 to each other. The wings 140 of adjacent ampules 100 are connected to each other by a folding portion 141 that is relatively thinner than the wings 140.

With the introduction of the wings 140 connected by the folding portion 141, one ampule set 100S consisting of a plurality of ampules 100 can be transported alone and stored in the shape of a flat plate, and the ampules 100 can be bent radially and arranged in a circle using the folding portion 141 when the ampules 100 are mounted on the cassette body 200. In addition, although not illustrated in the drawings, when one roll-shaped ampule product is manufactured by connecting a plurality of ampule sets 100S to each other, the folding portion 141 may be cut to correspond to the ampule sets 100S according to the specification of the cassette body 200.

The cassette body 200 is composed of the upper body 210 to which the upper ends (the neck portions 120) of the ampules 100 are fixed, the lower body 220 to which the lower ends (the fitting portions 130) of the ampules 100 are fixed, and coupling members 230 which couple the bodies 210 and 220 to each other.

The upper and lower bodies 210 and 220 have circular plate shapes corresponding to each other. The upper body 210 includes a plurality of fitting holes 211 whose lower surfaces are open along a rim, which are arranged radially, and to which the neck portions 120 are fitted. The lower body 220 includes the fitting groove 221 which is formed in a ring shape to have an upper surface open along a rim and into which the fitting portions 130 are fitted.

Due to the fitting holes 211 and the fitting groove 221, the ampule set 100S mounted on the cassette body 200 is disposed and fixed upright in a circle along a circumferential direction of the bodies 210 and 220.

In this case, the fitting holes 211 are separated from each other, and the number of the fitting holes 211 is not limited, However, the number of the fitting holes 211 may correspond one-to-one to the number of the ampules 100 to be mounted (that is, the number of the neck portions 120 to be fitted) in view of the width of the body portion 110, more preferably, may be n times as many as the number of the ampules 100 to be mounted so that the ampule set 100S can be mounted in any direction.

The sterilant cassette C containing the hydrogen peroxide is horizontally mounted in the mounting unit 12 of the plasma sterilizer S in a state where the ampules 100 stand upright with the neck portions 120 facing upward, and the upper body 210 or the lower body 220 is coupled to a rotary medium (e.g., a rotary shaft connected to an electric motor) of the mounting unit 12 to rotate the sterilant cassette C containing the hydrogen peroxide.

Therefore, when the sterilant injector 30 advances forward for sterilant injection, the needle unit 31 pierces the body portion 110 of one ampule 100 to inject the sterilant inside the storage space 111 by a fixed quantity. When the sterilant in one ampule 100 is exhausted, the sterilant injector 30 retreats backward. Then, the cassette body 200 rotates at a predetermined angle to position a new ampule 100 in the direction of the sterilant injector 30. After this, the sterilant injector 30 advances forward again to inject the sterilant.

The neck portions 120 and the fitting portions 130 function not only as fixing media for fixing and coupling the ampules 100 between the bodies 210 and 220 but also as support media for supporting the body portion 110 of each ampule 100 against the advancing pressure of the needle unit 31 when the needle unit 31 pierces the body portion 110 by the forward movement of the sterilant injector 30.

In this case, various known methods are applicable to the rotation of the sterilant cassette C containing the hydrogen peroxide in the mounting unit 12 of the sterilizer.

Each of the upper and lower bodies 210 and 220 is provided with one or more coupling holes 212 or 222 at the center, and the coupling members 230 are made to stand upright as their both ends are inserted into the upper and lower coupling holes 212 and 222 to couple the bodies 210 and 220 together.

In the drawings, a pair of coupling holes 212 or 222 are provided in each body 210 or 220, and two coupling members 230 are coupled to the coupling holes 212 and 222. The number of the coupling holes 212 and 222 and the number of the coupling members 230 are not limited.

General bolts may be used as the coupling members 230. However, since the ampules 100 are fixed upright between the upper and lower bodies 210 and 220 in the present disclosure, an appropriate gap may be maintained between the upper and lower bodies 210 and 222 in view of the height of the ampules 100 (more strictly, the total height of the body portion 110 and the neck portion 120).

Therefore, each of the coupling members 230 of the present disclosure includes a gap maintaining tube 231 disposed upright between the upper body 210 and the lower body 220 to maintain a gap between the bodies 210 and 220 and a bolt member 232 penetrating the upper and lower bodies 210 and 220 and the gap maintaining tube 231.

The gap maintaining tube 231 is a tubular body penetrated by a through hole 231a in a longitudinal direction. The gap maintaining tube 231 is formed to a length corresponding to the height of the body portions 110 in view of depths to which the neck portions 120 and the fitting portions 130 are inserted into the fitting holes 211 and the fitting groove 221, respectively.

The bolt member 232 includes a body 232A formed to be longer than the gap maintaining tube 231 and having threads on an outer circumferential surface and a head 232B provided at an end of the body portion 232A.

In this case, either the upper coupling holes 212 or the lower coupling holes 222 do not have corresponding threads on their inner circumferential surfaces while penetrating both upper and lower surfaces of the body to accommodate the heads 232B when the bolt members 232 are coupled. The other coupling holes on the opposite side have corresponding threads, to which the threads of the bodies 232A are screwed, on their inner circumferential surfaces while penetrating the upper or lower surface of the body so that the bodies 232A of the bolt members 232 can be screwed.

By the coupling members 230, each composed of the gap maintaining tube 231 and the bolt member 232, the upper and lower bodies 210 and 220 of the cassette body 200 can be firmly coupled to each other while maintaining a gap corresponding to the height of the body portions 110.

Although not illustrated in the drawings, the head 232B of each of the bolt members 232 is provided with a tool fastening portion (e.g., a "+" or "-"-shaped driver groove or a hexagonal wrench groove) for tightening or releasing the bolt member 232 using a tool.

Although the respective neck portions 120 of the ampules 100 are fitted and fixed into the fitting holes 211 in the present disclosure, a predetermined gap should be formed between the neck portions 120 and the fitting holes 211 in order to easily insert or separate the neck portions 120 into or from the fitting grooves 211.

In this case, since the needle unit 31 advances forward to pierce the body portions 110 and inject the sterilant as described above, if the gap is formed between the neck portions 120 and the fitting grooves 211, the neck portions 120 (and/or the body portions 110) may be twisted or pushed by the advancing pressure of the needle unit 31.

The twisting or pushing of the neck portions 120 (and/or the body portions 110) may cause the piercing failure of the needle unit 31 or inaccurate piercing of the needle unit 31, which is better than the case where the neck portions 120 inserted into the fitting holes 211 are firmly fixed (due to their thin flat plate structure, the fitting portions 130 can be easily inserted into and removed from the fitting groove 221 even without the formation of a gap between them and are relatively less twisted or pushed than the neck portions 120).

In this regard, the present disclosure includes a fixing medium 240 for fixing the neck portions 120 of the ampules 100 to the fitting holes 211.

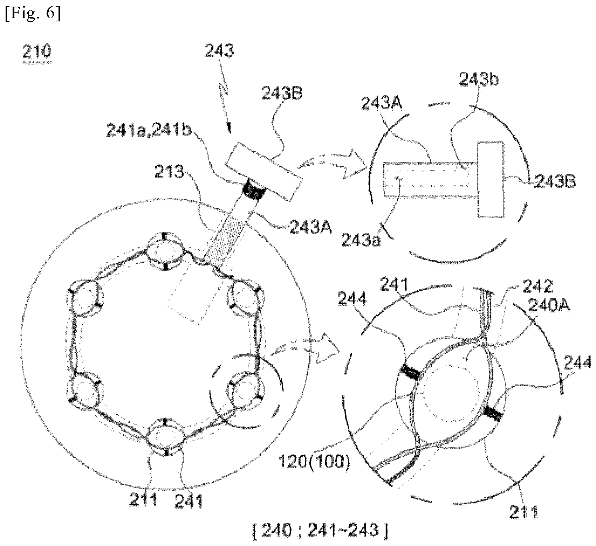

Referring to FIG. 6 which is a schematic plan view of the upper body 210, the fixing medium 240 includes a first fixing wire 241 and a second fixing wire 242 which pass through all of the fitting holes 211 of the upper body 210 and are connected in a zigzag fashion in opposite directions to each other and a tightening member 243 which is coupled to both the first and second fixing wires 241 and 242 exposed through one side surface of the upper body 210 and is tightened or loosened to decrease or increase lengths of the first and second fixing wires 241 and 242.

The first fixing wire 241 passes through the circularly arranged adjacent fitting holes 211 in a zigzag fashion, and the second fixing wire 242 passes through the adjacent fitting holes 211 in a zigzag fashion in a direction opposite to that of the first fixing wire 241. A circular pressing portion 240A is formed in each fitting hole 211 by the first and second fixing wires 241 and 242 disposed in opposite directions, and each of the neck portions 120 inserted into the fitting holes 211 is inserted into the circular pressing portion 240A.

Both ends of the first and second fixing wires 241 and 242 are exposed to the outside through a screw hole 213 formed in one side surface of the upper body 210.

The tightening member 243 may be tightened or loosened in the screw hole 213 of the upper body 210 and includes a tubular body 243A which is screwed to the screw hole 213 and a grip portion 243B which is connected to an end of the tubular body 243A. The tubular body 243A includes a passage hole 243a formed along the central longitudinal direction and an exposure hole 243b formed laterally and connected to the through hole 243a.

The tubular body 243A of the tightening member 243 is screwed to the screw hole 213. Here, both ends of the first and second fixing wires 241 and 242 pass through the passage hole 243a to be exposed through the exposure hole 243b and to be fixed and coupled to an outer circumference of the tubular body 243A or to the grip portion 243B.

Therefore, when a user tightens the tightening member 243 by rotating the grip portion 243B in one direction in a state where the upper and lower bodies 210 and 220 are coupled to the ampule set 100S, the first and second fixing wires 241 and 242 exposed through the exposure hole 243b are wound around the outer circumference of the tubular body 243A. Accordingly, as the lengths of the first and second fixing wires 241 and 242 passing through the fitting holes 211 in a zigzag fashion are reduced, the circular pressing portion 240A disposed in each of the fitting holes 211 contracts to fix the neck portion 120 inserted into the fitting hole 211 by pressing both side surfaces of the neck portion 120. When the user loosens the tightening member 243 by rotating the grip portion 243B in the other direction, the first and second fixing wires 241 and 242 wound around the tubular body 243A return to their original state to widen the circular pressing portion 240A, thereby separating the neck portion 120 from the fitting hole 211.

Here, if the first and second fixing wires 241 and 242 are wound when the screwing direction of the tightening member 243 is a general thread direction (i.e., right-hand threads), the winding of the fixing wires may be interfered by the reducing space between the grip portion 243B and the upper body 210. Therefore, the threads of the tubular body 243A may be formed in a direction (i.e., left-hand threads) opposite to the general thread direction, so that the fixing wires are wound around the tubular body 243A when the tightening member 243 is loosened by being rotated in a clockwise direction which is generally a tightening direction.

In this case, to facilitate the winding of the first and second fixing wires 241 and 242 and the return to the original state when the first and second fixing wires 241 and 242 are unwound, the first and second fixing wires 241 and 242 may include twisted portions 241a and 242a which are formed by helically twisting part of tip end portions of the first and second fixing wires 241 and 242 and are wound around or unwound from the outer circumference of the tubular body 243A.

The first and second fixing wires 241 and 242 are made of a steel wire having certain rigidity and elasticity (e.g., austenitic stainless steel such as SUS304) so that it is possible to return to the original state in which the circular twisted portions 241a and 242a are widened when the tightening member 243 is loosened.

In addition, if the first and second fixing wires 241 and 242 are too thin or made of a too soft material, the first and second fixing wires 241 and 242 that form the circular pressing portion 240A in each fitting hole 211 may sag or may not return to their original state to widen the circular pressing portion 240A when the tightening member 243 is loosened.

Thus, the present disclosure includes a pair of elastic bodies 244, each having an end connected to an inner circumference of each fitting hole 211 and the other end connected to the first fixing wire 241 or the second fixing wire 242 that forms the circular pressing portion 240A so as to exert an elastic force in a direction in which the circular pressing portion 240A is widened.

If the elastic bodies 244 configured as described above are introduced between each fitting hole 211 and the first and second fixing wires 241 and 242, when the fixing wires are unwound from the tightening member 243, the circular pressing portion 240A is widened by the elastic force of the elastic bodies 244 to return the first and second fixing wires 241 and 242 to their original state. At the same time, since the circular pressing portion 240A is kept widened by the elastic force of the elastic bodies 244, it is possible to secure a space through which each neck portion 120 can pass.

While the present disclosure has been described with reference to the accompanying drawings, mainly regarding a sterilant cassette containing hydrogen peroxide having specific shape and configuration, those skilled in the art will appreciate that various modifications, additions and substitutions are possible, without departing from the scope and spirit of the invention as disclosed in the accompanying claims.

## Claims

1. A plasma sterilizer comprising:
a light emitting diode (LED) monitoring system which is mounted in the plasma sterilizer S for sterilizing objects in a vacuum and indicates the progress of sterilization using the on/off, color change or both of a plurality of LED lamps 11a;
a sterilant cassette C containing hydrogen peroxide which is mounted in a mounting unit;
a vacuum chamber 20 in which the objects are received; and
a sterilant injector 30 which sucks a sterilant from ampules of the sterilant cassette C containing the hydrogen peroxide and supplies the sucked sterilant to the vacuum chamber 20,
wherein the sterilant cassette C containing the hydrogen peroxide comprises a plurality of ampules 100 in which the sterilant is stored and a cassette body 200 which is comprised of an upper body 210 to which upper ends of the ampules 100 are fixed and a lower body 220 to which lower ends of the ampules 100 are fixed so as to fix the ampules 100 upright between the upper body 210 and the lower body 220,
wherein each of the ampules 100 is comprised of a body portion 110 which has a sterilant storage space, a neck portion 120 which has a reducing width and is connected to an upper side of the body portion 110 and a fitting portion 130 which is connected to a bottom surface of the body portion 100, the upper body 210 and the lower body 220 have circular plate shapes corresponding to each other, the upper body 210 comprises a plurality of fitting holes 211 whose lower surfaces are open along a rim, which are arranged radially and to which the neck portions 120 are fitted, the lower body 220 comprises a fitting groove 221 which is formed in a ring shape to have an upper surface open along a rim and into which the fitting portions 130 are fitted, and the sterilant cassette C containing the hydrogen peroxide further comprises a fixing medium 240 which can fix the neck portions 120 of the ampules 100 to the fitting grooves 211,
wherein the fixing medium 240 comprises a first fixing wire 241 and a second fixing wire 242 which pass through all of the fitting holes 211 of the upper body 210 and are connected in a zigzag fashion in opposite directions to each other and a tightening member 243 which is coupled to both the first and second fixing wires 241 and 242 exposed through one side surface of the upper body 210 and is tightened or loosened to decrease or increase lengths of the first and second fixing wires 241 and 242,
wherein the first fixing wire 241 passes through the circularly arranged adjacent fitting holes 211 in a zigzag fashion, the second fixing wire 242 passes through the adjacent fitting holes 211 in a zigzag fashion in a direction opposite to that of the first fixing wire 241, a circular pressing portion 240A is formed in each fitting hole 211 by the first and second fixing wires 241 and 242 disposed in opposite directions, each of the neck portions 120 is inserted into the circular pressing portion 240A, both ends of the first and second fixing wires 241 and 242 are exposed to the outside through a screw hole 213 formed in one side surface of the upper body 210, and the tightening member 243 is tightened or loosened in the screw hole 213 of the upper body 210 and is comprised of a tubular body 243A which is screwed to the screw hole 213 and a grip portion 243B which is connected to an end of the tubular body 243A,
wherein the tubular body 243A comprises a passage hole 243a formed along a central longitudinal direction and an exposure hole 243b formed laterally and connected to the through hole 243a.

2. The plasma sterilizer of claim 1, wherein threads of the tubular body 243A are left-hand threads opposite to a general thread direction so that the first and second fixing wires 241 and 242 are wound around the tubular body 243A when the tightening member 243 is rotated clockwise, the first and second fixing wires 241 and 242 comprise twisted portions 241a and 242a which are formed by spirally twisting part of tip end portions of the first and second fixing wires 241 and 242 and wound around or unwound from an outer circumference of the tubular body 243A to facilitate the winding of the first and second fixing wires 241 and 242 and the return to the original state when the first and second fixing wires 241 and 242 are unwound, the first and second fixing wires 241 and 242 are made of a steel wire having rigidity and elasticity so that it is possible to return to the original state in which the twisted portions 241a and 242a are widened when the tightening member 243 is loosened, and the fixing medium 240 further comprises a pair of elastic bodies 244, each having an end connected to an inner circumference of each fitting hole 211 and the other end connected to the first fixing wire 241 or the second fixing wire 242 so as to exert an elastic force in a direction in which the circular pressing portion 240A is widened.
